# EUROPEAN PATENT SPECIFICATION

(11) **EP 2 953 711 B1**
(45) Date of publication and mention of the grant of the patent: **18.07.2018**
(21) Application number: 14705941.4
(22) Date of filing: 07.02.2014
(51) Int. Cl.: B01F 17/00

(54) **DISPERSANTS**
DISPERSIONSMITTEL
AGENTS DISPERSANTS

(30) Priority: 11.02.2013 US 201361763253 P
(43) Date of publication of application: 16.12.2015
(73) Proprietor: Croda, Inc., Edison, NJ 08837 (US)
(72) Inventor: HUGHES, John Ernest, Lincoln University, PA 19352 (US); MCNAMEE, William Harry, Claymont, DE 19703 (US); CHEN, Xin, Hockessin, DE 19707 (US)
(74) Representative: Craven, Ian
(86) International application number: PCT/US2014/015205
(87) International publication number: WO 2014/124196

(56) References cited:
- WO-A1-03/106010
- WO-A1-2007/034199
- GB-A- 1 301 138
- US-A- 2 389 680
- US-A- 3 623 979
- US-A1- 2010 323 112
- US-A1- 2011 034 593

## Description

This application is related to and claims the benefit of U.S. Provisional Application No. 61/763,253 entitled "DISPERSANTS" filed on February 11, 2013, the contents of which are incorporated herein by reference.

### FIELD OF THE INVENTION

The present invention relates to dispersants for particulate inorganic or organic compounds, in particular pigments, a process of making said dispersants and compositions comprising said dispersant and particulate inorganic or organic compounds.

### BACKGROUND OF THE INVENTION

Dispersants for pigments are well known. Solvent borne coating compositions which contain pigments often require addition of dispersants to effectively disperse and stabilise the composition, especially where particularly fine particle sizes are used. One existing pigment dispersant which is typically used is based upon polycaprolactone/dodecanoic acid/polyetherimide. This existing dispersant is sold and used for automotive and industrial solvent based paints.

Polycaprolactone/dodecanoic acid/polyetherimide dispersants are typically solid dispersant and therefore difficult to work with. Additionally, other similar dispersants often require a synergist to help bond the dispersant on to the pigment, and therefore it would be advantageous to use a dispersant which does not require a synergist.

In particular, dispersants for pigments, such as carbon blacks, are used in coatings in the automotive industry. These coatings are applied to metal. It is well known to those skilled in the coating composition arts that carbon black pigmentation of solvent-reduced coating compositions is one of the most difficult of pigmentations to perform to consistent and high standards of colour development.

It has been generally held that a predominant problem for carbon black pigmentation of solvent-reduced coating compositions (such as acrylic enamels, nitrocellulose lacquers, and alkyd enamels) resides in the difficulty of fully realising the potential for black colour development of this class of pigments as compared to the relative ease by which development can generally be achieved with other pigments conventionally employed in the art.

Similar chemistries of compounds are used in other technical fields. These include US 2,389,680 which discloses treatment of bitumen with amino alkyl amides of carboxylic acid, where the alkyl group is a C₁₀ to C₂₃ saturated or unsaturated chain. The treatment of the bitumen results in improved adhesivity for bonding mineral aggregates.

US 6,982,078 discloses dimer amidopropyl poly-quaternary compounds. These compounds are based on use of C₃₆ dimer diacid and are disclosed for use in barrier products for personal care applications.

EP 1073511 discloses diamine neutralised compound and a carboxylic acid. The polymeric difunctional cationic compounds are disclosed for use as emollients and conditioners in cosmetic, personal care and household products.

The present invention therefore seeks to provide dispersants which can exhibit good dispersant properties for particulate inorganic or organic compounds especially pigments, in compositions. The present invention also seeks to provide a dispersant having good colour development of pigments, including carbon black pigments, in solvent-reduced protective and decorative coating compositions. In particular, the present invention seeks to provide a dispersant with said properties allowing for less solvent to be used in a composition whilst having mechanical and other physical properties at levels comparable to prior dispersants and compositions.

The present invention further seeks to provide a method of making the dispersant having improved properties, and also seeks to provide the use of said dispersant in a composition.

### SUMMARY OF THE INVENTION

According to a first aspect of the present invention there is provided a composition comprising at least one particulate inorganic or organic compound, and a dispersant, said dispersant comprising a residue of a dimer fatty acid and/or trimer fatty acid having at least one carboxylic acid group derivatised to provide a terminal secondary or tertiary amine.

According to a second aspect of the present invention there is provided a dispersant formed by reaction of a dimer fatty acid and/or trimer fatty acid and a secondary or tertiary amine comprising compound.

According to a third aspect of the present invention there is provided the use of a dispersant, according to the first aspect or as formed by the second aspect, for dispersing a particulate inorganic or organic compound in a composition.

### DETAILED DESCRIPTION OF THE INVENTION

It has been found that dimer fatty acid and/or trimer fatty acid residues having at least one carboxylic acid group derivatised to provide a terminal secondary or tertiary amine can be used as a dispersant for particulate inorganic or organic compound, and in particular pigments, in compositions, and that this dispersant provides good dispersancy properties. Due to the improved dispersancy said compositions may comprise less solvent than would be required for existing dispersants, or can allow for higher pigment loading when using the same amounts of dispersant as are used with existing dispersants. These advantageous effects have been found particularly when using the dispersant with pigments such as carbon blacks which are used for automotive coatings.

As used herein, the terms 'for example,' 'for instance,' 'such as,' or 'including' are meant to introduce examples that further clarify more general subject matter. Unless otherwise specified, these examples are provided only as an aid for understanding the applications illustrated in the present disclosure, and are not meant to be limiting in any fashion.

It will be understood that, when describing the number of carbon atoms in a substituent group (e.g. 'C₁ to C₆ alkyl'), the number refers to the total number of carbon atoms present in the substituent group, including any present in any branched groups. Additionally, when describing the number of carbon atoms in, for example fatty acids, this refers to the total number of carbon atoms including the one at the carboxylic acid, and any present in any branch groups.

The dispersant may preferably be selected from compounds represented by general formula (I) and/or (II):

[(R²)(R¹)N-L¹-]ₘ-D-[-L²-N(R³)(R⁴)]ₙ (I)

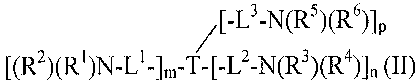

wherein
D represents a dimer fatty acid;
T represents a trimer fatty acid;
L¹, L², and L³ each independently represent a linking group comprising between 1 and 10 carbon atoms;
R¹, R², R³, R⁴, R⁵, and R⁶ each independently represent hydrogen or a C₁ to C₆ alkyl;
at least one of R¹ and R² represents a C₁ to C₆ alkyl, at least one of R³ and R⁴ represents a C₁ to C₆ alkyl, and at least one of R⁵ and R⁶ represents a C₁ to C₆ alkyl;
for formula (I) the sum of m and n is either 1, or 2; and
for formula (II) the sum of m, n, and p is 1, 2, or 3.

The term dimer fatty acids (also referred to as dimer diacids or dimer fatty diacid) is well known in the art, and refers to the dimerisation products of mono- or polyunsaturated fatty acids and/or esters thereof. The related term trimer fatty acid similarly refers to trimerisation products of mono- or polyunsaturated fatty acids and/or esters thereof.

Dimer fatty acids are described T. E. Breuer, 'Dimer Acids', in J. I. Kroschwitz (ed.), Kirk-Othmer Encyclopedia of Chemical Technology, 4th Ed., Wily, New York, 1993, Vol. 8, pp. 223-237. They are prepared by polymerising fatty acids under pressure, and then removing most of the unreacted fatty acid starting materials by distillation. The final product usually contains some small amounts of mono fatty acid and trimer fatty acids, but is mostly made up of dimer fatty acids. The resultant product can be prepared with various proportions of the different fatty acids as desired.

The ratio of dimer fatty acids to trimer fatty acids can be varied, by modifying the processing conditions and/or the unsaturated fatty acid feedstock. The dimer fatty acid may be isolated in substantially pure form from the product mixture, using purification techniques known in the art, or alternatively a mixture of dimer fatty acid and trimer fatty acid may be employed.

The dimer fatty acids used in the present invention are preferably derived from the dimerisation products of C₁₀ to C₃₀ fatty acids, more preferably C₁₂ to C₂₄ fatty acids, particularly C₁₄ to C₂₂ fatty acids, further preferably C₁₆ to C₂₀ fatty acids, and especially C₁₈ fatty acids. Thus, the resulting dimer fatty acids preferably comprise in the range from 20 to 60, more preferably 24 to 48, particularly 28 to 44, further preferably 32 to 42, and especially 36 carbon atoms.

The fatty acids, from which the dimer fatty acids are derived, may be selected from linear or branched unsaturated fatty acids. The unsaturated fatty acids may be selected from fatty acids having either a cis/trans configuration, and may have one or more than one unsaturated double bonds.

Preferably, the fatty acids used are linear monounsaturated fatty acids.

Suitable dimer fatty acids are preferably derived from (i.e. are the dimer equivalents of) the dimerisation products of oleic acid, linoleic acid, linolenic acid, palmitoleic acid, or elaidic acid. In particular, suitable dimer fatty acids are derived from oleic acid.

The dimer fatty acids may be dimerisation products of unsaturated fatty acid mixtures obtained from the hydrolysis of natural fats and oils, e.g. sunflower oil, soybean oil, olive oil, rapeseed oil, cottonseed oil, or tall oil.

The molecular weight (weight average) of the dimer fatty acid is preferably in the range from 450 to 690, more preferably 500 to 640, particularly 530 to 610, and especially 550 to 590.

In addition to the dimer fatty acids, dimerisation usually results in varying amounts of trimer fatty acids (so-called "trimer"), oligomeric fatty acids, and residues of monomeric fatty acids (so-called "monomer"), or esters thereof, being present. The amount of monomer can, for example, be reduced by distillation.

Similarly, the optional trimer fatty acids are preferably derived from the trimerisation products of the materials mentioned with regard to the dimer fatty acids, and are preferably trimers of C₁₀ to C₃₀, more preferably C₁₂ to C₂₄, particularly C₁₄ to C₂₂, further preferably C₁₆ to C₂₀ fatty acids, and especially C₁₈ fatty acids. Thus, the trimer fatty acids preferably contain in the range from 30 to 90, more preferably 36 to 72, particularly 42 to 66, further preferably 48 to 60, and especially 54 carbon atoms.

The molecular weight (weight average) of the trimer fatty acids is preferably in the range from 750 to 950, more preferably 790 to 910, particularly 810 to 890, and especially 830 to 870.

In one embodiment of the present invention, tetramer fatty acids and higher oligomers (hereinafter both referred to as oligomeric acids) are formed during production of the dimer fatty acid. Such oligomeric acids may therefore also be present in the dimer fatty acids used in the present invention, in combination with trimer fatty acids and/or dimer fatty acids and/or monomeric fatty monoacids.

The oligomeric acids are preferably oligomers, containing 4 or more units derived from C₁₀ to C₃₀, more preferably C₁₂ to C₂₄, particularly C₁₄ to C₂₂, and especially C₁₈ fatty acids. The molecular weight (weight average) of the oligomeric acid is suitably greater than 1,000, preferably in the range from 1,200 to 1,800, more preferably 1,300 to 1,700, particularly 1,400 to 1,600, and especially 1,400 to 1,550.

In one embodiment the dispersant may be formed using only dimer diacid or trimer triacid. In an alternative preferred embodiment, dispersants formed from mixtures of dimer diacid and trimer triacid may be used.

Dimer fatty diols and trimer fatty triols may also be used in forming the dispersants. Dimer fatty acids and trimer fatty acids are discussed herein, and dimer fatty diols and trimer fatty triols may be formed by hydrogenation of the corresponding dimer/trimer fatty acids. The same preferences detailed for the dimer/trimer fatty acids apply to the corresponding dimer fatty diol trimer fatty triol.

The dimer fatty acid used in the present invention preferably may have a dimer fatty acid (or dimer) content of greater than 60 wt.%, more preferably greater than 70 wt.%, particularly greater than 80 wt.%, and especially greater than 85 wt.%. In addition, particularly preferred dimer fatty acids may have a trimer fatty acid (or trimer) content of less than 40 wt.%, more preferably less than 30 wt.%, particularly less than 20 wt.%, and especially less than 15 wt.%.

In an alternative embodiment, the dimer fatty acid preferably has a dimer fatty acid (or dimer) content in the range from 70 wt.% to 99 wt.%, such as from 70 to 96 wt.%. This may be applicable in particular for two component or cross-linked systems.

In a further embodiment, the dimer content of the dimer fatty acid may be in the range from 90 wt.% to 99 wt.%, and the trimer fatty acid content may be less than 1 wt.%.

Furthermore, the dimer and or trimer fatty acid preferably comprises less than 10 wt.%, more preferably less than 6 wt.%, particularly less than 4 wt.%, and especially less than 3.5 wt.% of mono fatty monoacid (or monomer).

Particularly preferred mixtures of dimer fatty diacid and trimer fatty triacid may be comprised of in the range;
from 70 to 90 wt.% dimer fatty diacid, 10 to 30 wt.% trimer fatty triacid;
from 85 to 99.5 wt.% dimer fatty diacid, 0.1 to 10 wt.% trimer fatty triacid; or
from 15 to 35 wt.% dimer fatty diacid, 50 to 90 wt.% trimer fatty triacid,
with any remaining balance of the mixture being made up of monomer fatty acid or other oligomers.

All of the above weight percentage values are based on the total weight of polymerised fatty acids and mono fatty acids present.

L¹, L², and L³ each represent a linking group present between the carboxylic acid functional group of the dimer diacid or trimer triacid, and the secondary or tertiary terminal amine.

Said linking groups L¹, L², and L³ each independently comprise between 1 and 10 carbon atoms. Preferably, said linking groups may each be an alkyl or substituted alkyl comprising between 1 and 6 carbon atoms. More preferably, said linking groups may each be an alkyl or substituted alkyl comprising between 1 and 4 carbon atoms. Most preferably, said linking groups may each be an alkyl or optionally substituted alkyl comprising either 2 or 3 carbon atoms.

The linking group may each independently be straight chain or branched moieties.

The alkyl lower alkyls may be optionally substituted with hydroxy, fluoro, chloro, bromo, iodo, nitro, OR⁷, SR⁸, NR⁹R¹⁰, aryl, or het groups, wherein R⁷ to R¹⁰ each independently represent hydrogen, or C₁ to C₃ lower alkyl.

The linking groups each independently may preferably be terminally substituted with a primary amine or hydroxyl group

Examples of linking groups may be independently selected from methyl, ethyl, n-propyl, isopropyl, n-butyl, isobutyl, sec-butyl, tert-butyl, 2 methyl-butyl, or the like.

The groups R¹, R², R³, R⁴, R⁵, and R⁶ each independently represent hydrogen or a C₁ to C₆ alkyl. Additionally, at least two of R¹ and R² represent a C₁ to C₆ alkyl, at least two of R³ and R⁴ represent a C₁ to C₆ alkyl, and at least two of R⁵ and R⁶ represent a C₁ to C₆ alkyl. Preferably, all groups R¹, R², R³, R⁴, R⁵, and R⁶ each independently represent a C₁ to C₆ alkyl.

The term 'C₁ to C₆ alkyl' as used herein, unless otherwise defined, refers to saturated hydrocarbon radicals being straight chain, or branched moieties containing 1 to 6 carbon atoms.

Preferably, the groups R¹, R², R³, R⁴, R⁵, and R⁶ each independently represent a C₁ to C₄ alkyl. More preferably, the groups R¹, R², R³, R⁴, R⁵, and R⁶ each independently represent a C₁ to C₃ alkyl.

The groups R¹, R², R³, R⁴, R⁵, and R⁶ may be independently selected from methyl, ethyl, n-propyl, isopropyl, n-butyl, isobutyl, sec-butyl, tert-butyl, 2 methyl-butyl, pentyl, hexyl, or the like. More preferably, the groups R¹, R², R³, R⁴, R⁵, and R⁶ may be independently selected from methyl, ethyl, n-propyl, isopropyl, n-butyl, isobutyl, sec-butyl, or tert-butyl. Most preferably, the groups R¹, R², R³, R⁴, R⁵, and R⁶ may be independently selected from methyl or ethyl.

In particular the linking group and the secondary or tertiary amine may be provided in the same reactant compound. Said compound may be selected from an amine, diamine, alcohol, or an amine alcohol. Preferably, said compound is selected from a diamine or an amine alcohol.

In this embodiment, it is envisaged that the groups [(R²)(R¹)N-L¹-]ₘ-, [(R⁴)(R³)N-L²-]ₙ-, and [(R⁶)(R⁵)N-L³-]ₚ- may all be identical as they may be derived from the same reactant compound.

Suitable amine alcohols may be selected from n,n-dimethylethanolamine (DMEA), diethylaminoethanol (DEEA), diethanolamine (DEA), and triethanolamine (TEA).

Suitable diamines may be selected from, dimethylaminopropylamine (DMAPA), or trimethylethylenediamine.

Preferably, said diamines or amine alcohols are selected from n,n-dimethylethanolamine (DMEA), diethylaminoethanol (DEEA), or dimethylaminopropylamine (DMAPA).

The resulting dispersant formed by reaction of a dimer fatty diacid may be mono-substituted with the terminal tertiary or secondary amine comprising compound. Said dispersant may therefore be mono-substituted where the sum of values m and n is equal to 1. Alternatively, said dispersant may be di-substituted where the sum of values m and n is equal to 2.

Where the dispersant is formed from trimer fatty triacid, the sum of the values of m, n, and p may be equal to 1, 2, or 3 for mono-, di-, or tri-substituted respectively.

It is envisaged that the dispersant may comprise a mixture of the mono and d- substituted compounds. Where trimer triacid is present the dispersant may also comprise tri-substituted compounds.

The dispersant may comprise at least 50 wt.% of di-substituted compounds. More preferably, at least 60 wt.%. Further preferably, in the range from 70 wt.% to 98 wt.%.

The dispersant may comprise less than 30 wt.% of mono-substituted compounds. More preferably, less than 20 wt.%. Further preferably, in the range from 10 wt.% to 1 wt.%.

In the embodiment where the dispersant is formed from, at least in part, trimer triacid, the dispersant may comprise less than 40 wt.% of mono-substituted compounds. More preferably, less than 30 wt.%. Further preferably, in the range from 5 wt.% to 20 wt.%.

The ratio of mono to di substituted dispersant compound may be altered as desired, and is well within the remit of the relevant skilled person. Alteration of the mono:di substituent ratio may be effected by, for example, use of protecting groups changing the ratio of dimer diacid to diamine/amine alcohol present in the reaction mixture.

The dispersant may be synthesised by any method, with suitable method being well known to the skilled person.

A suitable example of a synthesis method may comprise the steps of;
adding a reducing agent to the dimer fatty diacid and/or trimer fatty triacid,
heating the reaction mixture,
adding the secondary or tertiary amine comprising compound,
placing the reaction mixture under a partial vacuum,
removing any excess secondary or tertiary amine comprising compound,
cooling the reaction mixture, and
discharging the dispersant product.

The dispersant formed preferably has a hydroxyl value, or alkaline value where no hydroxyl is present, (measured as described herein) in the range from 200 to 100, more preferably 170 to 110, particularly 160 to 120, and especially 150 to 130 mgKOH/g.

In addition, the dispersant preferably has an acid value (measured as described herein) of less than 10 mgKOH/g, more preferably less than 8 mgKOH/g, particularly less than 6 mgKOH/g, and especially less than 5 mgKOH/g.

The dispersant formed preferably has a water content less than 0.8%, more preferably less than 0.5%, particularly less than 0.3%, and especially less than 0.15%.

The ratio of secondary or tertiary amine comprising compound to diacid and/or trimer fatty triacid to during synthesis is in the range from 1.2-4:1 respectively. Preferably, in the range from 1.5-3:1. Most preferably, in the range from 1.8-2.5:1

The particulate inorganic or organic compounds present in the composition may be selected from any suitable particulate inorganic or organic compound. In particular, said particulate inorganic or organic compounds are pigments comprised in a coating composition.

The particulate inorganic or organic compound may have a particle size in the range from 5 to 200 nm. More preferably, in the range from 8 to 150 nm. Most preferably, 10 to 100 nm.

A non-limiting example of a suitable pigment includes carbon black. Examples of suitable carbon black pigments include those manufactured by Cabot Corporation, Columbian Chemicals Company, and Orion Engineered LLC.

Other examples of inorganic pigments include metal oxides, such as the oxides of iron, zinc, copper, manganese, or titanium.

The pigment may preferably be carbon black. Selection of the specific carbon blacks or carbon black pigments may be made by the skilled person according to the coating requirements. Preferably, the particle size for the carbon black pigment is in the range from 10 to 50 nm, more preferably in the range from 10 to 25 nm.

Suitable organic or inorganic pigment particles may be selected from black pigment particles, yellow pigment particles, magenta pigment particles, red pigment particles, violet pigment particles, cyan pigment particles, blue pigment particles, green pigment particles, orange pigment particles, brown pigment particles or white pigment particles.

The amount of pigment present in the coating composition may preferably be in the range from about 0.1 wt.% to 5.0 wt.% of the total composition. More preferably, in the range from 0.5 to 2.0% by weight.

In particular where the pigment is carbon black, these values of pigment present may be used to produce film thicknesses of up to 50 nm. In the case of higher film thicknesses, a larger amount of carbon black of up to 20 wt.%, relative to the substrate, may be used.

The composition may also include additional ingredients such as performance enhancing or modifying agents. The performance enhancing or modifying agents, by way of example, may be selected from flexibilisers, toughening agents/particles, core shell rubbers, flame retardants, wetting agents, dyes, flame retardants, plasticisers, UV absorbers, anti-fungal compounds, fillers, viscosity modifiers/flow control agents, tackifiers, stabilisers, and inhibitors.

The dispersant of the present invention is used in a composition for dispersion of particulate inorganic or organic compounds. In particular, said composition may be a colourant composition.

As used herein, the term 'colourant composition,' unless otherwise defined, refers to compositions which can impart a colour to a surface or substrate to which they are applied thereon. The colourant compositions will therefore comprise a pigment or mixture of pigments which are dispersed throughout the composition by the dispersant. Examples of colourant compositions include ink toners, coatings, paints, and radar absorbants. In particular, colourant composition may be coatings. Preferably, said coatings are coatings for use in the automotive industry.

The dispersant may be used in colourant compositions which are coating compositions. Coating compositions can be a single-layer coating or a primer (or primer-basecoat combined). Coatings can be on metal or other suitable substrates.

The dispersant may particularly find use as a dispersant for carbon black pigments in high performance applications, for example in automotive coating systems.

The colourant composition may be an acrylic paint or resin based composition.

The colourant composition may comprise surfactants, and these may be selected from anionic, cationic, or non-ionic surfactants. If present, the colourant composition may comprise in the range from 0.01 to 20 wt.% surfactants. More preferably, in the range from 0.05 wt.% to 15 wt.%. Most preferably, in the range from 0.1 to 3% by weight.

The use of the dispersant of the present invention may allow for a colourant composition where the amount of dispersant required to disperse the pigment is less than existing prior art dispersants.

Preferably the ratio of dispersant to particulate inorganic or organic compound in the composition is less than 0.06:1, as a ratio of percentage weights. More preferably, the ratio of dispersant to particulate inorganic or organic compound is less than 0.05:1. Further preferably, less than 0.04:1. Most preferably, less than 0.03:1.

The ratio of dispersant to particulate inorganic or organic compound may remain substantially the same in the composition when initially formulated, or if added to, for example, an acrylic resin paint.

The level of dispersant of the present invention required to reduce a specified level of pigment may therefore be reduced by at least 25%, preferably more than 50% when compared to the level required for existing dispersants.

Without wishing to be unduly bound by theory, it has been found that the benefits of the invention may be conferred due to the presence of the secondary or tertiary terminal amine groups which helps with adhesion to negatively charged particulate inorganic or organic compounds. Additionally, it is believed that the use of the described dispersant reduces particulate agglomeration.

All of the features described herein may be combined with any of the above aspects, in any combination.

In order that the present invention may be more readily understood, reference will now be made, by way of example, to the following description.

It will be understood that all tests and physical properties listed have been determined at atmospheric pressure and room temperature (i.e. 20°C), unless otherwise stated herein, or unless otherwise stated in the referenced test methods and procedures.

### Test Methods

▪ The acid value is defined as the number of mg of potassium hydroxide required to neutralise the free fatty acids in 1 g of sample, and was measured by direct titration with a standard potassium hydroxide solution.
▪ The alkali (or hydroxyl) value is defined as the number of mg of potassium hydroxide equivalent to the hydroxyl content of 1g of sample, and was measured by acetylation followed by hydrolysation of excess acetic anhydride. The acetic acid formed was subsequently titrated with an ethanolic potassium hydroxide solution.

### Synthesis of Dispersants

A series of dispersants were prepared as summarised in Table 1. Compounds as used in the following synthesis examples are identified as follows:

### Acids

A1 - mixture of 95-98 wt.% dimer of C₁₈ fatty acid, 2-4 wt.% trimer of C₁₈ fatty acid
A2 - mixture of 75-80 wt.% dimer of C₁₈ fatty acid, 18-22 wt.% trimer of C₁₈ fatty acid
A3 - mixture of 70-80 wt.% trimer of C₁₈ fatty acid, 20-30 wt.% dimer of C₁₈ fatty acid

Acid mixtures A1-A3 were all obtained from Croda.

### Amines

DMAPA - dimethylaminopropylamine
DMEA - dimethylethanolamine
DEEA - diethylaminoethanol

**Table 1. - Dispersants synthesised**

| **Synthesis Example** | **Acid** | **Amine** | **Compound Made** |
|---|---|---|---|
| S1 | A1 | DMAPA | DMAPA Dimeramide |
| S2 | A2 | DMAPA | DMAPA Dimeramide |
| S3 | A3 | DMAPA | DMAPA Trimeramide |
| S4 | A2 | DMEA | DMEA Dimerate |
| S5 | A2 | DEEA | DEEA Dimerate |

### Synthesis of Dispersant S1

Into a clean 2-L 4-neck reactor flask equipped with distillation column, 1002g (3.5 equivalents based on carboxylic acid) of acid A1 was added, and 2.5g of hypophosphorous acid (50% solution). With nitrogen sparge and agitation on, the mixture was heated to 160°C. 376g DMAPA was added in to the reactor flask over 3 hours with condensation water removed from the reaction. After all the DMAPA was added, the reaction mixture was heated to 180°C over a period of 1 hour, and then held at that temperature for 1 hour. A partial vacuum at 700 Torr was then applied, and the reaction held 180°C for additional 2 hours.

Once the total acid value had dropped to less than 6, the vacuum was reduced to 10-20 Torr. The reaction mixture was held for 2 hours to distill off any excess DMAPA. The reaction mixture was cooled to less than 80 °C, and then discharged from the reaction flask.

The product obtained (DMAPA dimeramide) was a clear liquid at room temperature, and had total acid value less than 5 and alkali value of 178. The structure of the product was confirmed with NMR methods.

### Synthesis of Dispersant S2

Dispersant S2 was synthesised in the same way as S1, and used acid A2 instead of A1. The resulting product was a clear liquid at room temperature, and had total acid value less than 5 and alkali value of 154.

### Synthesis of Dispersant S3

Dispersant S3 was synthesised in the same way as S1, and used acid A3 instead of A1. The resulting product was a clear liquid at room temperature, and had total acid value less than 5 and alkali value of 175.

### Synthesis of Dispersant S4

Dispersant S4 was synthesised in the same way as S1, and used acid A2 instead of Al, and DMEA instead of DMAPA. The resulting product was a clear liquid at room temperature, and had total acid value less than 7 and alkali value of 144.

### Synthesis of Dispersant S5

Dispersant S5 was synthesised in the same way as S1, and used acid A2 instead of A1, and DEEA instead of DMAPA. The resulting product was a clear liquid at room temperature, and had total acid value less than 5 and alkali value of 154.

### Dispersant Formulation Examples

A number of dispersant formulations were prepared using the dispersants S1-S5. Compounds as used in the following examples are identified as follows:
▪ Solsperse 24000 (existing dispersant available from Lubrizol) - polycaprolactone/dodecanoic acid/polyetherimide. This was included as a comparative example and does do not fall with the scope of this invention.
▪ Dispersants S1 to S5, as synthesised herein, were used.
▪ Monarch 1300 Carbon Black pigment
▪ Aromatic 100 - light aromatic naphtha, predominately C₈ - C₁₀ hydrocarbons.

### Carbon Black Dispersions

Dispersion formulations D1 and D2 were made to determine performance of the dispersants with carbon black pigments. The formulations were made on a Dispermat LC with a Norstone 1.625" Cowles-Type blade. The formulations were mixed up in a Qorpack 16 oz glass jar.

**Table 2. - Dispersion formulations**

| **Compound** | **D1** | **D2** | **D3** |
|---|---|---|---|
| Resin binder | 49.6g | 49.6g | 49.6g |
| n-pentyl propionate | 16.75g | 16.75g | 16.75g |
| S1 | 103.0g | --- | --- |
| S2 | --- | --- | 103.0g |
| S3 | --- | 103.0g | --- |
| Monarch 1300 Carbon Black | 102.44g | 102.44g | 102.44g |
| Aromatic 100 | 189.48g | 189.48g | 189.48g |

Formulations D1, D2, and D3 were formed to determine the efficacy of the dispersants in a resin system that used Aromatic 100 as the solvent. It was found that dispersion formulations D1, D2, and D3 dispersed the pigment with no issues. These samples were then tested further.

The dispersion formulation (D3) comprising S2 was tested against Solsperse 24000 (comparative examples) to determine viscosity over time as the samples were dispersed under high speed (4000 rpm) for 60 minutes, with samples removed every 15 minutes. Whilst mixing at high speeds the dispersant was tested to determine shear effects and if the dispersant broke down and caused the black dispersion to obtain a high enough viscosity that the samples gelled.

**Table 3. Viscosity versus time of paint**

| **Sample Time (minutes)** | **Viscosity of D3 Dispersed Paint (cps)** | **Viscosity of Solsperse 24000 Dispersed Paint (cps)** |
|---|---|---|
| 15 | 1750 | 4550 |
| 30 | 1870 | 5850 |
| 45 | 2050 | 8300 |
| 60 | 2380 | 12000 |

The results in Table 3 show the difference in viscosity between the S2 based dispersion formulation and the Solsperse 24000 comparative example formulation over time. It can be seen that no gelation occurred with the S2 dispersant and viscosity remained relatively low. The S2 dispersant therefore allows for more fluid pigment dispersion than the Solsperse 24000 when used at the same levels of actives.

Based on this information, a reduction by 50% actives loading was done with the S2 dispersant to compare with the Solsperse 24000. After dispersing for 30 minutes at 4,000 rpm the samples were then run through the M100 Eiger Mill for four runs. The results indicated that at 50% actives concentration, as compared to the Solsperse 24000, the S2 dispersant was as, if not more, effective at dispersing high-performance carbon black pigments.

The Solsperse 24000 dispersed sample showed a rapid increase in viscosity from around 4500 cps at the 15 minute mark to 12000 cps at the 60 minute mark. The S2 dispersant, used at the same actives level as the Solsperse 24000, showed only minor increases in viscosity from 1800 cps at 15 minutes, to 2100 cps at 60 minutes.

**Table 4. Percentage Colour Strength Difference**

| **Solsperse 24000 Dispersed Paint** | **S2 Based Dispersed Paint (used at 50% loading)** |
|---|---|
| 100% | 103.5% |

Table 4 shows the difference in percentage colour strength between the S2 dispersed carbon black paint and the Solsperse 24000 carbon black paint, as taken on a BYK Gardner TSC II colorimeter. The S2 dispersant, when used at 50% actives level, has better colour development after milling compared to the Solsperse 24000 at 100% actives level.

Further dispersion formulations were made in a resin system comprising a 60% solution of Laropal A81 (available from BASF) mixed with propylene glycol monomethyl ether acetate (PM acetate). The paint system used was the Deltron DBX 1689 basecoat converter.

**Table 5. Dispersants Used at 25% Actives Level as Compared to Solsperse 24000**

| **Chemical** | **D4** | **D5** | **D6** |
|---|---|---|---|
| Laropal A81 60% | 31.87g | 31.87g | 31.87g |
| S5 | 0.317g | --- | --- |
| S4 | --- | 0.317g | --- |
| S1 | --- | --- | 0.317g |
| PM Acetate | 42.32g | 42.32g | 42.32g |
| Monarch 1300 Carbon Black | 25.49g | 25.49g | 25.49g |

Table 5 shows the dispersions that were made with the differing versions of the dimer amide. These dispersions were then put into a paint formulation to be tested to determine the performance properties.

**Table 6. Paint Formulations using Dispersions made in Table 5**

| **Component** | **P1** | **P2** | **P3** |
|---|---|---|---|
| Deltron DBX 1689 | 47.0g | 47.0g | 47.0g |
| D4 | 3.0g | --- | --- |
| D5 | --- | 3.0g | --- |
| D6 | --- | --- | 3.0g |

The formulations shown in Table 6 were used for measuring percentage colour strength versus time. Samples were taken every 15 minutes for 60 minutes and added to the Deltron DBX 1689 Basecoat Converter. Once four samples were done, they were then placed on a Red Devil 1410 Paint Shaker for 60 minutes. The samples were then removed from the paint shaker and drawdowns were performed against both the dispersed paint and the Solsperse 24000 control.

The viscosity measurements were taken using the 25% actives samples and compared to the Solsperse 24000 control. Results are shown in Table 7.

**Table 7. Viscosity of Dispersions at 25% Actives Level as Compared to Solsperse 24000**

| **Sample Time (minutes)** | **Viscosity of Solsperse 24000 (cps)** | **Viscosity of S2 Based Dispersion at 25% Actives (cps)** |
|---|---|---|
| 15 | 4550 | 1120 |
| 30 | 5850 | 1290 |
| 45 | 8300 | 1050 |
| 60 | 12000 | 1420 |

Table 7 shows the various viscosity measurements using a Brookfield RVT Synchro-Electric Viscometer using an RV Spindle 4 at 20 rpm. All of the dispersants of the present invention showed much lower viscosities throughout the entire 60 minutes when compared to the Solsperse 24000.

For the final milling of the samples, the following formulations were tested.

**Table 8. Samples for Eiger Milling at 50% Actives Level Compared to Solsperse 24000**

| **Component** | **D7** | **D8** |
|---|---|---|
| Laropal A81 60% | 157.85g | 157.85g |
| S2 | 3.14g | --- |
| Solsperse 24000 | --- | 6.3g |
| Xylene | --- | 24.5g |
| PM Acetate | 208.03g | 191.96g |
| Monarch 1300 Carbon Black Pigment | 126.25g | 126.25g |

The paint dispersions that were prepared for Eiger Milling is shown in Table 8. The samples were dispersed at 4000 rpm on the Dispermat then run through the Eiger Mill for 4 passes. After Eiger Milling the samples were tested by viscosity and drawdowns to determine performance properties. Table 8 shows the differing formulations used to test the new dimer amide dispersant verses Solsperse 24000, which is considered the industry standard.

Once the pigment dispersion was made, a small sample was then used for testing in a paint to determine color strength. The paint that the samples were placed into was PPG's Deltron DBX 1689 basecoat converter. This cured within 1 hour and was clear, which allowed for measurements to be taken on the pigment's ability to provide color strength and not any tinting from the paint.

**Table 9. Viscosities of Eiger Milled Samples**

| **Sample** | **Viscosity (cps)** |
|---|---|
| D7 | 3640 |
| D8 | 4790 |

The viscosities of the Eiger Milled samples are shown in Table 9 as compared to each other. The Solsperse 24000 had the highest viscosity of 4,790 cps and was unable to be poured from the container. The other sample were pourable and fluid.

**Table 10. Percentage Colour Strength of Eiger Milled Samples**

| **Sample** | **% Strength** |
|---|---|
| D7 | 101.5 |
| D8 | 100 |

Table 10 shows the percentage colour strength of the sample as compared to the Solsperse 24000 control (D8).

Following the milling of the samples, paints were made up from the pigment dispersions. These paints were then tested to determine the performance properties versus the Solsperse 24000 control.

**Table 11. Paint Formulations from Pigment Dispersions**

| **Component** | **P4** | **P5** |
|---|---|---|
| Deltron DBX 1689 | 47.0g | 47.0g |
| D7 | 3.0g | --- |
| D8 | --- | 3.0g |

The paint samples were mixed for 60 minutes on the Red Devil 1410 Paint Shaker. After mixing, they were set aside for 2 hours, and then tested by drawdown method to determine percentage colour strength by the BYK Gardner TSC II colorimeter.

**Table 12. Percentage Colour Strength of Paints Using Monarch 1300 Carbon Black Pigment**

| **Sample** | **% Strength** |
|---|---|
| P4 | 95.9 |
| P5 | 100 |

The paint made with the dispersant of the present invention compared favourably with that made with the Solsperse 24000, as shown in Table 12.

### Magenta Pigment Dispersions

Testing was undertaken using the dispersant S2 with a magenta pigment to determine if the dimer amides could be used to disperse other pigments than the high performance carbon blacks.

The magenta pigment used was Cinquasia magenta pigments available from BASF.

**Table 13. Paint Binder Formulation of S2 Based Dispersion and Solsperse 24000**

| **Component** | **M1** | **M2** |
|---|---|---|
| Laropal A81 60% | 31.6g | 31.6g |
| Solsperse 24000 | 1.2g | --- |
| Xylene | 4.9g | --- |
| S2 | --- | 0.6g |
| PM Acetate | 42.3g | 37.8g |
| Cinquasia Magenta Pigment | 20g | 20g |

The formulations shown in Table 13 were then mixed on the Dispermat for 60 minutes with sampling every 15 minutes. The samples were then placed in the Deltron DBX 1689 Basecoat Converter for mixing and further testing. The paint formulations made are shown in Table 14.

**Table 14. Paint formulations using dispersion formulations M1 and M2**

| **Component** | **P6** | **P7** |
|---|---|---|
| Deltron DBX 1689 | 47.0g | 47.0g |
| M1 | 3.0g | --- |
| M2 | --- | 3.0g |

The paint formulations P6 and P7 were each then placed on a Red Devil Classic paint shaker and shaken for 60 minutes. After which samples were placed on BYK byko-charts and tested using a 3ml drawdown bar. The drawn down samples were allowed to dry then tested on a BYK Gardner TSC II colorimeter. The colorimeter tested the colour strength of the paint systems using S2 dispersant compared to the paint system using the comparative example of Solsperse 24000.

Another series was done using the Eiger Mill after dispersing for 30 minutes at 4,000 rpms. A total of 4 runs were done with each sample through the Eiger Mill. After which the pigment dispersions were then put into a paint formulation as seen in Table 14.

**Table 15. Percentage colour strength of Cinquasia magenta pigmented paints**

| **Minutes** | **Solsperse 24000 Colour Strength** | **S2 Dispersant Strength (50%)** |
|---|---|---|
| 15 | 31.4 | 38.6 |
| 30 | 52.2 | 68.5 |
| 45 | 73.9 | 103.5 |
| 60 | 100 | 139.8 |

Table 15 shows the percentage colour strength performance of the magenta paints made with different dispersants. The S2 dispersant based formulations, while being at 50% less active level than the Solsperse 24000, showed better colour strength and coverage ability when used in the same paint formulation. This indicates that the S2 dispersant is a much better dispersant than the Solsperse 24000, which is currently considered a premier dispersant.

It is to be understood that the invention is not to be limited to the details of the above embodiments, which are described by way of example only. Many variations are possible.

## Claims

1. A composition comprising at least one particulate inorganic or organic compound, and a dispersant, said dispersant comprising a residue of a dimer fatty acid and/or trimer fatty acid having at least one carboxylic acid group derivatised by reaction with n,n-dimethylethanolamine (DMEA), diethylaminoethanol (DEEA), trimethylethylenediamine or dimethylaminopropylamine (DMAPA) to provide a terminal secondary or tertiary amine.

2. A composition as claimed in claim 1 wherein the ratio of dispersant to particulate inorganic or organic compound in the composition is less than 0.06:1, as a ratio of percentage weights.

3. A composition as claimed in any preceding claim wherein the at least one particulate inorganic or organic compound is a pigment.

4. A composition as claimed in any preceding claim wherein the composition is a coating composition.

5. A dispersant formed by reaction of a dimer fatty acid and/or trimer fatty acid and n,n-dimethylethanolamine (DMEA), diethylaminoethanol (DEEA), trimethylethylenediamine or dimethylaminopropylamine (DMAPA).

6. A colourant composition comprising at least one particulate inorganic or organic compound which is a pigment, and a dispersant as claimed in claim 5.

7. Use of a dispersant according to any of claims 1 to 5 for dispersing a particulate inorganic or organic compound in a composition.

8. Use of a dispersant as claimed in claim 7 wherein the particulate inorganic or organic compound is a pigment.

## Patentansprüche

1. Zusammensetzung, umfassend mindestens eine teilchenförmige anorganische oder organische Verbindung und ein Dispergiermittel, wobei das Dispergiermittel einen Rest einer Dimerfettsäure oder Trimerfettsäure mit mindestens einer zur Bereitstellung eines endständigen sekundären oder tertiären Amins durch Umsetzung mit N,N-Dimethylethanolamin (DMEA), Diethylaminoethanol (DEEA), Trimethylethylendiamin oder Dimethylaminopropylamin (DMAPA) derivatisierten Carbonsäuregruppe aufweist.

2. Zusammensetzung nach Anspruch 1, wobei das Verhältnis von Dispergiermittel zu teilchenförmiger anorganischer oder organischer Verbindung in der Zusammensetzung weniger als 0,06:1 als Gewichtsprozentverhältnis beträgt.

3. Zusammensetzung nach einem der vorhergehenden Ansprüche, wobei es sich bei der mindestens einen teilchenförmigen anorganischen oder organischen Verbindung um ein Pigment handelt.

4. Zusammensetzung nach einem der vorhergehenden Ansprüche, wobei es sich bei der Zusammensetzung um eine Beschichtungszusammensetzung handelt.

5. Dispergiermittel, gebildet durch Umsetzung einer Dimerfettsäure oder Trimerfettsäure und N,N-Dimethylethanolamin (DMEA), Diethylaminoethanol (DEEA), Trimethylethylendiamin oder Dimethylaminopropylamin (DMAPA).

6. Farbmittelzusammensetzung, umfassend mindestens eine teilchenförmige anorganische oder organische Verbindung, bei der es sich um ein Pigment handelt, und ein Dispergiermittel nach Anspruch 5.

7. Verwendung eines Dispergiermittels nach einem der Ansprüche 1 bis 5 zum Dispergieren einer teilchenförmigen anorganischen oder organischen Verbindung in einer Zusammensetzung.

8. Verwendung eines Dispergiermittels nach Anspruch 7, wobei es sich bei der teilchenförmigen anorganischen oder organischen Verbindung um ein Pigment handelt.

## Revendications

1. Composition comprenant au moins un composé organique ou inorganique particulaire, et un agent de dispersion, ledit agent de dispersion comprenant un reste d'un acide gras dimère et/ou d'un acide gras trimère ayant au moins un groupement acide carboxylique dérivatisé par une réaction avec de la N,N-diméthyléthanolamine (DMEA), du diéthylaminoéthanol (DEEA), de la triméthyléthylène-diamine ou de la diméthylaminopropylamine (DMAPA) afin de fournir une amine secondaire ou tertiaire terminale.

2. Composition selon la revendication 1, dans laquelle le rapport de l'agent de dispersion au composé organique ou inorganique particulaire dans la composition est inférieur à 0,06:1, comme rapport de pourcentages pondéraux.

3. Composition selon l'une quelconque des revendications précédentes, dans laquelle le au moins un composé organique ou inorganique particulaire est un pigment.

4. Composition selon l'une quelconque des revendications précédentes, où la composition est une composition de revêtement.

5. Agent de dispersion formé par la réaction d'un acide gras dimère et/ou d'un acide gras trimère avec de la N,N-diméthyléthanolamine (DMEA), du diéthylaminoéthanol (DEEA), de la triméthyléthylènediamine ou de la diméthylaminopropylamine (DMAPA).

6. Composition de colorant comprenant au moins un composé organique ou inorganique particulaire qui est un pigment, et un agent de dispersion selon la revendication 5.

7. Utilisation d'un agent de dispersion selon l'une quelconque des revendications 1 à 5, pour la dispersion d'un composé organique ou inorganique particulaire dans une composition.

8. Utilisation d'un agent de dispersion selon la revendication 7, dans laquelle le composé organique ou inorganique particulaire est un pigment.
